# EUROPEAN PATENT APPLICATION

(11) **EP 2 810 689 A1**
(43) Date of publication of application: **10.12.2014**
(21) Application number: 13170763.0
(22) Date of filing: 06.06.2013
(51) Int. Cl.: A61N 1/36, A61N 1/05

(54) **A system for planning and/or providing a therapy for neural applications**

(71) Applicant: Sapiens Steering Brain Stimulation B.V., 5656 AE Eindhoven (NL)
(72) Inventor: Martens, Hubert Cécile François, 5611 ND Eindhoven (NL); Decré, Michel Marcel Jose, 5654 NC Eindhoven (NL); Åström, Mattias, 5509 KD Veldhoven (NL); Nijlunsing, Rutger, 5507 LP Veldhoven (NL)
(74) Representative: Prinz & Partner mbB

(57) **Abstract**

The present invention relates to a system (10) for planning and/or providing a therapy for neural applications, especially for neurostimulation and/or neurorecording applications, comprising at least one lead (300), the lead (300) having a plurality of electrodes (132), at least one electrode (132) being capable to form an active contact (134) with a first potential, wherein the system (10) comprises at least one electrode potential adjusting means (400) which is configured such that at least one selected electrode (136) at (a) varying distance(s) from the active contact (134) may be provided with a second potential different from the first potential of the active contact (134) for the purpose of focusing the stimulation field (F) provided by the active contact (134). Furthermore, the present invention relates to a system for neural applications (100) and to a method for focusing the stimulation field provided by an active contact (134) of a lead (300).

## Description

The present invention relates to a system for planning and/or providing a therapy for neural applications, especially a neurostimulation and/or neurorecording applications.

Implantable neurostimulation devices have been used for the past ten years to treat acute or chronic neurological conditions. Deep brain stimulation (DBS), the mild electrical stimulation of sub-cortical structures, belongs to this category of implantable devices, and has been shown to be therapeutically effective for Parkinson's disease, Dystonia, and Tremor. New applications of DBS in the domain of psychiatric disorders (obsessive compulsive disorder, depression) are being researched and show promising results. In existing systems, the probes are connected to an implantable current pulse generator.

Currently, systems are under development with more, smaller electrodes in a technology based on thin film manufacturing. These novel systems consist of a lead made from a thin film based on thin film technology, as e.g. described in WO 2010/055453 A1. The thin film leads are fixed on a stylet material to form a probe. These probes will have multiple electrode areas and will enhance the precision to address the appropriate target in the brain and relax the specification of positioning. Meanwhile, undesired side effects due to undesired stimulation of neighbouring areas can be minimized.

Leads that are based on thin film manufacturing are e.g. described by US 7,941,202 and have been used in research products in animal studies.

In existing systems, the DBS lead has e.g. four 1.5 mm-wide cylindrical electrodes at the distal end spaced by 0.5 mm or 1.5 mm. The diameter of the lead is 1.27 mm and the metal used for the electrodes and the interconnect wires is an alloy of platinum and iridium. The coiled interconnect wires are insulated individually by fluoropolymer coating and protected in an 80A urethane tubing. With such electrode design, the current distribution emanates uniformly around the circumference of the electrode, which leads to stimulation of all areas surrounding the electrode.

The lack of fine spatial control over field distributions implies that stimulation easily spreads into adjacent structures inducing adverse side-effects in about 30% of the patients. To overcome this problem, systems with high density electrode arrays are being developed, hence providing the ability to steer the stimulation field to the appropriate target.

The clinical benefit of DBS is largely dependent on the spatial distribution of the stimulation field in relation to brain anatomy. To maximize therapeutic benefits while avoiding unwanted side-effects, precise control over the stimulation field is essential.

During stimulation with existing DBS leads there is an option to use monopolar, bipolar, or even tripolar stimulation. Neurostimulator devices with steering brain stimulation capabilities can have a large number of electrode contacts (n > 10) to which electrical settings such as current sources or grounding can be applied. Stimulation may be considered monopolar when the distance between the anode and cathode is several times larger than the distance of the cathode to the stimulation target. During monopolar stimulation in homogeneous tissue the electric field is distributed roughly spherical similar to the field from a point source. When the anode is located close to the cathode the distribution of the field becomes more directed in the anode-cathode direction. As a result the field gets denser and neurons are more likely to be activated in this area due to a higher field gradient.

The mechanisms of DBS are unknown. However, it is hypothesized that polarization (de- and/or hyperpolarization) of neural tissue is likely to play a prominent role for both suppression of clinical symptoms, as well as induction of stimulation-induced side-effects. In order to activate a neuron it has to be depolarized. Neurons are depolarized more easily close to the cathode than by the anode (about 3-7 times more depending on type of neuron, etc.).

Therefore, compared to monopolar stimulation the effect of bipolar stimulation is less spread of the electric field, a denser electric field between the anode and cathode, and more activated neurons close to the cathode. Bipolar stimulation is therefore used to focus the field to certain areas in cases when beneficial stimulation is not obtained during monopolar stimulation.

Due to the large number of electrode contacts of neurostimulator devices that allow asymmetrical steering of the stimulation field as provided by DBS system with lead having complex electrode arrays, it is not practical to have the user manually set individual electrode contacts to ground. Also, it is not trivial to apply grounding to appropriate contacts to get a certain amount of focus. Thus, the user is currently left without support of how to ground contacts when he wishes to focus the field.

It is therefore an object of the present invention, to improve a system for planning and/or providing a therapy for neural applications, especially a system for neurostimulation and/or neurorecording applications, in particular in that the user is supported when it is desired to focus the stimulation field provided by the electrodes of the lead and that the focusing is possible more intuitively and more accurately.

The above object is solved according to the present invention by a system for planning and/or providing a therapy for neural applications according to claim 1. Accordingly, a system for planning and/or providing a therapy for neural applications, especially for neurostimulation and/or neurorecording applications, comprising at least one lead, the lead having a plurality of electrodes, at least one electrode being capable to form an active contact with a first potential, wherein the system comprises at least one electrode potential adjusting means which is configured such that at least one selected electrode at (a) varying distance(s) from the active contact may be provided with a second potential different from the first potential of the active contact for the purpose of focusing the stimulation field provided by the active contact.

By this the advantage is achieved that a gradual focused stimulation field may be provided and that the user is supported when it is desired to focus the stimulation field provided by the electrodes of the lead and that the focusing is possible more intuitively and more accurately.

An active contact may be formed by one electrode. However, it is also possible that an active contact is formed by two or more electrodes. It is possible that such a plurality of electrodes consists at least partially of adjacent electrodes.

Especially, the lead may be a lead for neural applications, preferably a lead for a neurostimulation and/or neurorecording system. Such a neurostimulation and/or neurorecording system may be e.g. a DBS system.

The lead may e.g. comprise at least one thin film, whereby the thin film comprises a proximal end and a distal end, the lead further comprising a plurality of electrodes on the distal end of the thin film.

The thin film may include at least one electrically conductive layer, preferably made of a biocompatible material. The thin film may be assembled to the carrier and further processed to constitute the lead element. The thin film for a lead is preferably formed by a thin film product having a distal end, a cable with metal tracks and a proximal end. The distal end of the thin film may be forming a part of the distal end of the lead or substantially the distal end of the lead.

The distal end of the lead may be the end of the lead, which is in the implanted state of the lead the remote end of the lead with regard to the body surface area. In particular, in case of a lead for brain application, the distal end of the lead is the lower end of the lead, which is remote to the burr-hole of the skull, through which the lead is implanted.

There may be an Advanced Lead Can element, which may comprise electronic means to address the plurality of electrodes and at least one Advanced Lead Can connecting means. Further, the Advanced Lead Can element may be hermetically or substantially hermetically sealed and may comprise electronic means to address the plurality of electrodes on the distal end of the thin film, which is arranged at the distal end and next to the distal tip of the lead. The plurality of electrodes may comprise more than 5-10 electrodes, e.g. 16 or 32 electrodes or in preferred embodiments e.g. 64 electrodes or more. The electrodes may be arranged such that the electrodes are substantially evenly distributed arranged all over the distal end of the lead.

Furthermore, it is possible that the plurality of electrodes forms a complex geometrical array and/or that the plurality of electrodes is arranged circumferentially around at least one section of the lead, especially around a section next to the distal tip end of the lead.

By means of a complex array a stimulation field of any desired shape may be formed and may be adjusted according to the patient's needs. So, a suitable and accurate tailor-made stimulation field may be provided.

A complex array may be formed by a plurality of electrodes, which are arranged circumferentially around a section next to the distal tip end of the lead. The electrodes may be e.g. arranged non-planar and non-coaxial and likewise a leopard pattern and may form a regular array. Several electrodes may form at one level a ring around the lead and the next ring may be slightly displaced such that e.g. one electrode of the second ring is partially arranged within the gap between to electrodes of the first ring. So, there are rings of electrodes in radial direction of the lead and columns of electrodes in axial direction of the lead.

Additionally, it is possible that the electrode potential adjusting means is configured such that at least one selected electrode at (a) varying distance(s) from the active contact may be provided with a second potential different from the first potential of the active contact for the purpose of focusing the stimulation field provided by the active contact automatically and/or semi-automatically.

By an automatically and/or semi-automatically adjusting the advantage is achieved that the focusing of the stimulation field can be done very easy and more intuitively. A semi-automatic providing the electrodes at varying distances from the active contact with a different potential may be realized requiring a specific user input.

In particular, it is possible that, given an initial 'monopolar' stimulation setting, a concept and system can be provided that automatically applies grounding to appropriate electrode contacts to focus the stimulation field, where the amount of applied focus is controlled by the user with a scalar value e.g. a percentage. A large amount of focus corresponds to grounding of electrode contacts located close to the active electrode contacts, whereas a small amount focus corresponds to grounding of contacts located far away from the active electrode contacts.

Further, it is possible that the system is configured such that the second potential may be provided by providing and/or setting at least one electrode which is not a part of the active contact (to) a second polarity different from the first polarity of the active contact and/or by grounding at least one electrode which is not a part of the active contact.

Especially, the electrode potential adjusting means may be configured such that the second potential may be provided by providing and/or setting at least one electrode which is not a part of the active contact (to) a second polarity different from the first polarity of the active contact and/or by grounding at least one electrode which is not a part of the active contact.

For instance, groups of electrode contacts may be assigned to one or more 'cathodal' stimulation source(s). The user is then e.g. able to configure the stimulator device to deliver 'focused' stimulation, wherein during 'focused' stimulation groups of grounded electrodes are assigned adjacent to the first group of electrodes. A grading of the effect can be achieved by increasing or decreasing the distance of grounded contacts to the cathode electrode contact groups.

It is further possible that the electrode potential adjusting means is configured such that at least one electrode at (a) varying distance(s) from the active contact may be provided with at least one second potential different from the potential of the active contact for the purpose of focusing the stimulation field provided by the active contact in a first manner and that at least one electrode at (a) varying distance(s) from the active contact may be provided with at least one third potential different from the potential of the active contact for the purpose of focusing the stimulation field provided by the active contact in a second manner.

Moreover, it is possible that the second potential different from the potential of the active contact leads higher amount of focussing by setting at least one electrode next and/or adjacent from the active contact and the electrode(s) set for the second manner and that the third potential different from the potential of the active contact leads lower amount of focussing by setting at least one electrode further away from the active contact and the electrode(s) set for the first manner.

The electrodes next and/or adjacent from the active contact may be electrodes forming a ring around the active contact. The electrodes further away from the active contact may be electrodes which are e.g. not part of the ring around the active contact.

Additionally, it is possible that the electrode potential adjusting means is configured such that the Euclidian distance between at least one of the electrodes not belonging to the active contact is calculated, wherein especially the Euclidian distance between at least one of the electrodes not belonging to the active contact is calculated by means of a distance calculation.

By this, the advantage can be achieved that a mathematically based and thus exact determination of the electrodes to be changed regarding their potential can be provided.

Furthermore, it is possible that the electrode potential adjusting means is configured such that the total surface area of all electrodes with a potential different from the potential of the active contact is related to the amount of delivered current and the number of electrodes with a potential different from the potential of the active contact is adjusted according to a predetermined safety threshold value such that the charge density safety limit is not reached when focused stimulation is turned on.

By this, the advantage is achieved that the safety of the system may be increased. In particular, by this it is possible to assure that the charge density safety limit is not reached when focused stimulation is turned on, since the total surface area of e.g. all the grounded electrodes or electrodes with a different polarity is related to the amount of delivered current and the number of grounded electrodes or electrodes with a different polarity may be then adjusted accordingly.

Moreover, it is possible that the electrode potential adjusting means is configured such that the different potential may be gradually and/or stepwise decreased and/or increased.

Moreover, the present invention relates to a system for neural applications with the features of claim 9. Accordingly, a system for neural applications is provided, especially a system for neurostimulation and/or neurorecording applications, for instance a deep brain stimulation system. The system for neural applications comprises at least one system for planning and/or providing a therapy for neural applications according to one of claims 1 to 8.

Furthermore, the following method for planning and/or providing a therapy for neural applications, especially a neurostimulation and/or neurorecording applications with the features of claim 10 is disclosed.

Accordingly, the method for planning and/or providing a therapy for neural applications, especially a neurostimulation and/or neurorecording applications comprises at least the step of providing electrodes at varying distances from the active contact with a second potential different from the first potential of the active contact for the purpose of focusing the stimulation field provided by the active contact.

The lead comprises a plurality of electrodes and at least one electrode may be capable to form an active contact.

Within the method it is possible that the different potential as the active contact is provided by providing and/or setting at least one electrode which is not a part of the active contact (to) a second polarity different from the first polarity of the active contact and/or by grounding at least one electrode which is not a part of the active contact.

The method may further comprise the step that at least one electrode at (a) varying distance(s) from the active contact may be provided with at least one second potential different from the potential of the active contact for the purpose of focusing the stimulation field provided by the active contact in a first manner and that at least one electrode at (a) varying distance(s) from the active contact may be provided with at least one third potential different from the potential of the active contact for the purpose of focusing the stimulation field provided by the active contact in a second manner, wherein in particular the second potential different from the potential of the active contact leads to a higher amount of focussing by setting at least one electrode next and/or adjacent from the active contact and the electrode(s) set for the second manner and that the third potential different from the potential of the active contact leads to a lower amount of focussing by setting at least one electrode further away from the active contact and the electrode(s) set for the first manner.

It is further possible that the method further comprises the step that the Euclidian distance between at least one of the electrodes not belonging to the active contact is calculated, wherein especially the Euclidian distance between at least one of the electrodes not belonging to the active contact is calculated by means of a distance calculation.

Furthermore it is possible that the method further comprises the step that the total surface area of all electrodes with a potential different from the potential of the active contact is related to the amount of delivered current and the number of electrodes with a potential different from the potential of the active contact is adjusted according to a predetermined safety threshold value such that the charge density safety limit is not reached when focused stimulation is turned on.

The method may be conducted with at least one system for planning and/or providing a therapy for neural applications according to one of claims 1 to 8 and/or a system for neural applications according to claim 9.

It is possible that the above described method and the preferred embodiments thereto for planning and/or providing a therapy for neural applications are only used in-vitro or for testing and planning purposes only.

However, also it is also explicitly disclosed that the above described method and the preferred embodiments thereto for planning and/or providing a therapy for neural applications may be used for planning and/or providing a therapy for neural applications, especially a neurostimulation and/or neurorecording applications like DBS when the lead is implanted into the patient during therapy.

Further details and advantages of the present invention shall be described hereinafter with respect to the drawings:
- Fig. 1:: a schematical drawing of a neurostimulation system for deep brain stimulation (DBS);
- Fig. 2:: a further schematical drawing of a probe of a neurostimulation system for deep brain stimulation (DBS) and its components;
- Fig. 3:: a schematical drawing of a probe system according to the present invention;
- Fig. 4:: a schematical and abstract overview of an array of electrodes;
- Fig. 5:: a further schematical and abstract overview of an array of electrodes; and
- Fig. 6:: an overview of a possible embodiment of the system 10 according to the invention.

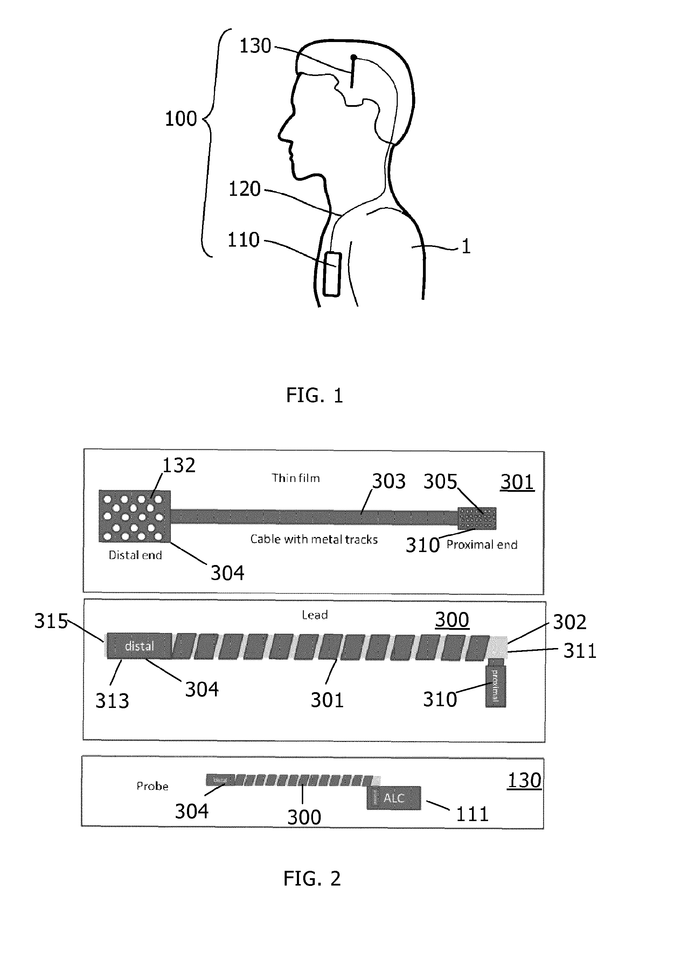

A possible embodiment of a neurostimulation system 100 for deep brain stimulation (DBS) is shown in Figure 1. The neurostimulation system 100 comprises at least a controller 110 that may be surgically implanted in the chest region of a patient 1, typically below the clavicle or in the abdominal region of a patient 1. The controller 110 can be adapted to supply the necessary voltage pulses. The typical DBS system 100 may further include an extension wire 120 connected to the controller 110 and running subcutaneously to the skull, preferably along the neck, where it terminates in a connector. A DBS lead arrangement 130 may be implanted in the brain tissue, e.g. through a burr-hole in the skull.

Figure 2 further illustrates a typical architecture for a Deep Brain Stimulation probe 130 that comprises a DBS lead 300 and an Advanced Lead Can element 111 comprising electronic means to address electrodes 132 on the distal end 304 of the thin film 301, which is arranged at the distal end 313 and next to the distal tip 315 of the DBS lead 300. The lead 300 comprises a carrier 302 for a thin film 301, said carrier 302 providing the mechanical configuration of the DBS lead 300 and the thin film 301. The thin film 301 may include at least one electrically conductive layer, preferably made of a biocompatible material. The thin film 301 is assembled to the carrier 302 and further processed to constitute the lead element 300. The thin film 301 for a lead is preferably formed by a thin film product having a distal end 304, a cable 303 with metal tracks and a proximal end 310. The proximal end 310 of the thin film 301 arranged at the proximal end 311 of the lead 300 is electrically connected to the Advanced Lead Can element 111. The Advanced Lead Can element 111 comprises the switch matrix of the DBS steering electronics. The distal end 304 comprises the electrodes 132 for the brain stimulation. The proximal end 310 comprises the interconnect contacts 305 for each metal line in the cable 303. The cable 303 comprises metal lines (not shown) to connect each distal electrodes 132 to a designated proximal contact 305.

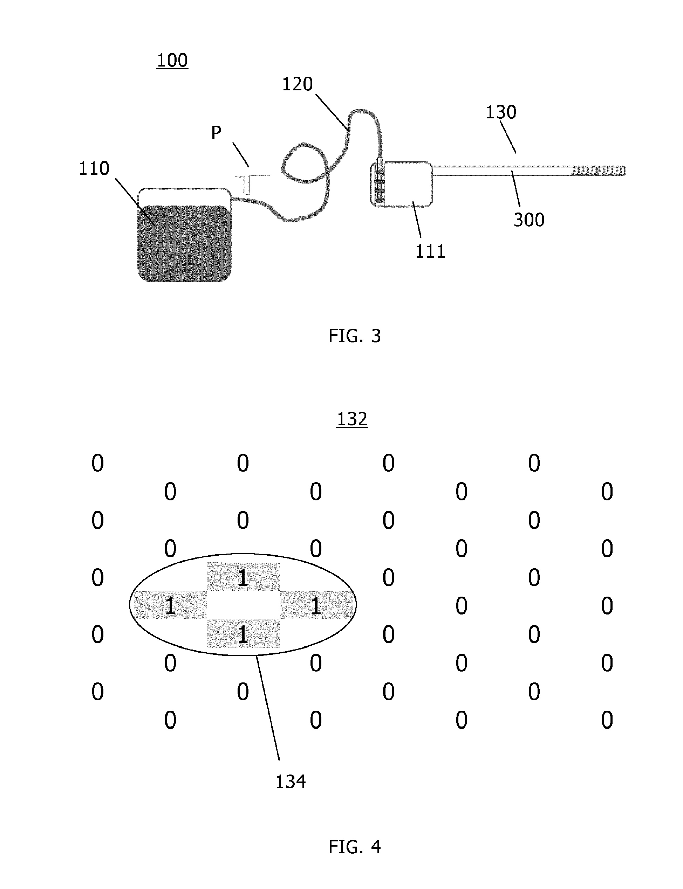

Figure 3 shows schematically and in greater detail an embodiment of a system 100 for brain applications, here for neurostimulation and/or neurorecording as a deep brain stimulation system 100 as shown in Figures 1 and 2. The probe system 100 comprises at least one probe 130 for brain applications with stimulation and/or recording electrodes 132, whereby e.g. 64 electrodes 132 can be provided on outer body surface at the distal end of the probe 130. By means of the extension wire 120 pulses P supplied by controller 110 can be transmitted to the Advanced Lead Can 111. The controller 110 can be an implantable pulse generator (IPG) 110.

Figure 4 shows a schematical and abstract overview of an (unfolded) array of electrodes 132, here 8 columns of electrodes 132 and 10 rows of electrodes 132.

The plurality of electrodes 132 forms a complex geometrical array and the plurality of electrodes 132 is arranged circumferentially around at least one section of the lead, here around a section next to the distal tip end of the lead 300.

Gradual focused stimulation can be achieved by grounding contacts of electrodes 132 at a varying distance from the active contact 134. The active contact 134 may be at least one (or also a plurality of several electrodes 132) which is active and which is "stimulating" the tissue next and/or adjacent to the electrode 132 and lead 300. The active contact 134 may be also named as cathode, i.e. at least one electrode 132 through which e.g. electric current flows out of the electrode.

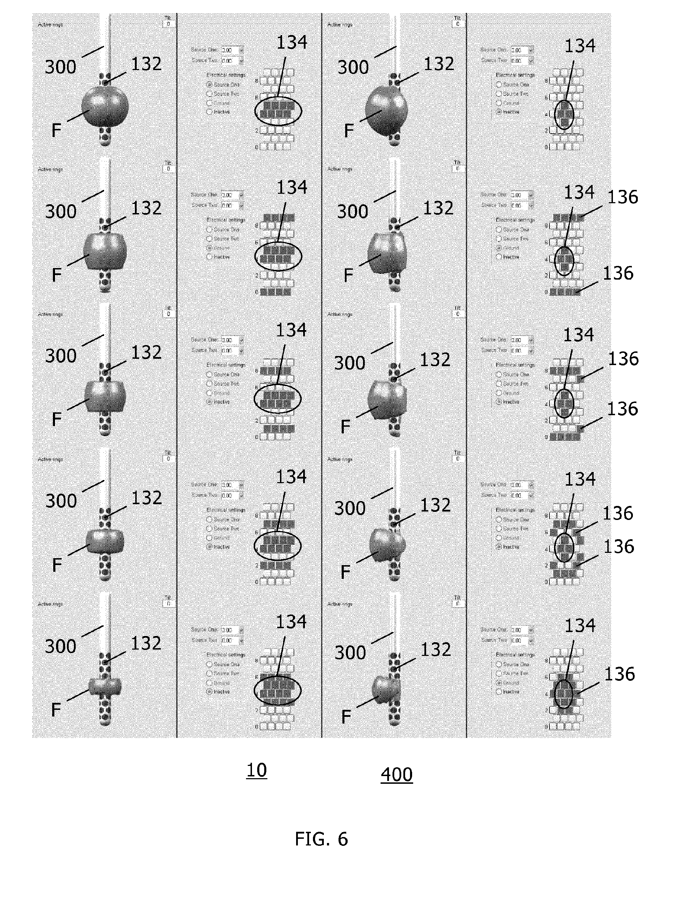

In order to obtain a strong focusing effect on the stimulation field F (see e.g. Figure 6) it is essential that the grounded contacts are located close to the active contact(s) 134, e.g. the cathode(s).

In the current concept, full focusing effect is generated by automatically grounding contacts adjacent to the active contact 134. When a lower amount of focus is desired, electrodes 132 further away from the active contact 134 are grounded, see Figure 4. In Figure 4, "1" refers to active electrodes 132 and form the active contact 134 and "0" refer to inactive electrodes.

In order to decide what contacts should be grounded to obtain a certain amount of focusing a distance transformation of all the active electrode contacts may be used.

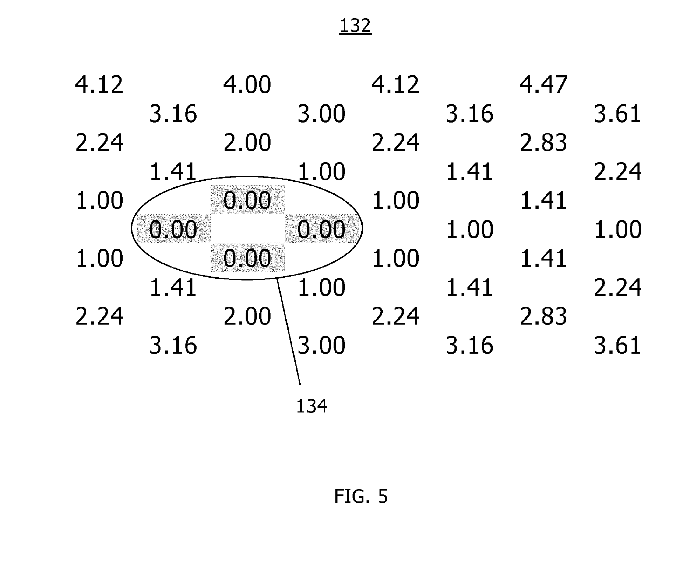

Figure 5 is a simple example of how the distance transformation is used.

The Euclidian distance between every electrode 132 to the active electrodes 132 forming the active contact 134 can then be calculated with a distance transformation.

The numbers representing each electrode 132 reflect the Euclidian distance to the active contact 134. In this example the distances are ranging from about 4 to 1. Thus, the amount of levels that could be used for gradual adjustment of the focusing is 4. During the lowest grade of focusing the contacts with a distance of about 4 would be grounded. During the highest grade of focusing the electrodes 132 with a distance of about 1 would be grounded.

In order to make sure that the charge density safety limit is not reached when focused stimulation is turned on, the total surface area of all the grounded electrodes 132 is related to the amount of delivered current, and the number of grounded electrodes 132 may be adjusted accordingly. If the number of electrodes 132 is low in comparison to the charge density safety limit, additional electrodes may be grounded. This might be needed since all the current that is delivered by the active contact 134 will also flow through the grounded electrodes 132.

In Figure 6 a first overview of a possible embodiment of the system 10 according to the invention is illustrated.

The system 10 is a system for planning and/or providing a therapy for neural applications, here a system 10 for neurostimulation and/or neurorecording applications, namely a system 10 for planning and providing deep brain stimulation therapy.

The system 10 comprises a lead 300 having a plurality of electrodes 132. The electrodes 132 are capable to provide at least one stimulation field F.

At least one electrode 132 is capable to form an active contact 134, wherein the system 10 comprises at least one electrode potential adjusting means 400 which is configured such that electrodes 132 at varying distances from the active contact 134 may be provided with a different potential as the active contact 134 for the purpose of focusing the stimulation field F provided by the active contact 134.

The plurality of electrodes 132 forms a complex geometrical array and the plurality of electrodes 132 is arranged circumferentially around at least one section of the lead, especially around a section next to the distal tip end of the lead 300.

The electrode potential adjusting means 400 is configured such that at least one selected electrode 136 at (a) varying distance(s) from the active contact 134 may be provided with a second potential different from the first potential of the active contact 134 for the purpose of focusing the stimulation field (F) provided by the active contact 134 automatically and/or semi-automatically. The different potential as the active contact 134 may be provided by providing and/or setting at least one electrode 132 which is not a part of the active contact 134 (to) a different polarity as the active contact 134 and/or by grounding at least one electrode 132 which is not a part of the active contact 134.

Further, the electrode potential adjusting means 400 is configured such that at least one electrode 136 at (a) varying distance(s) from the active contact 134 may be provided with at least one second potential different from the potential of the active contact 134 for the purpose of focusing the stimulation field provided by the active contact 134 in a first manner and that at least one electrode 136 at (a) varying distance(s) from the active contact 134 may be provided with at least one third potential different from the potential of the active contact 134 for the purpose of focusing the stimulation field provided by the active contact 134 in a second manner.

The second potential different from the potential of the active contact 134 leads higher amount of focussing by setting at least one electrode 136 next and/or adjacent from the active contact 134 and the electrode(s) 136 set for the second manner and that the third potential different from the potential of the active contact 134 leads lower amount of focussing by setting at least one electrode 136 further away from the active contact 134 and the electrode(s) 136 set for the first manner.

The electrodes next and/or adjacent from the active contact may be electrodes forming a ring around the active contact. The electrodes further away from the active contact may be electrodes which are e.g. not part of the ring around the active contact.

Moreover, the electrode potential adjusting means 400 is capable to calculate the Euclidian distance between at least one of the electrodes not belonging to the active contact 134, wherein especially the Euclidian distance between at least one of the electrodes not belonging to the active contact 134 is calculated by means of a distance calculation (see also e.g. Figure 5).

By this, the advantage can be achieved that a mathematically based and thus exact determination of the electrodes to be changed regarding their potential can be provided.

The total surface area of all electrodes 132, 136 with a potential different from the potential of the active contact 134 is related to the amount of delivered current and the number of electrodes 132, 136 with a potential different from the potential of the active contact 134 is adjusted according to a predetermined safety threshold value such that the charge density safety limit is not reached when focused stimulation is turned on.

By this, the advantage is achieved that the safety of the system may be increased. In particular, by this it is possible to assure that the charge density safety limit is not reached when focused stimulation is turned on, since the total surface area of e.g. all the grounded electrodes or electrodes with a different polarity is related to the amount of delivered current and the number of grounded electrodes or electrodes with a different polarity may be then adjusted accordingly.

The different potential may be gradually and/or stepwise decreased and/or increased.

As can been further see in Figure 6 in the far left column, the distribution of the stimulation fields F as produced by a neurostimulator device 100 with the lead 300 is visualized with cathodic electrical fields (e.g. in red), at an isolevel of 400 V/m. The focusing effect is gradually increased from top to bottom by grounding contacts closer to the cathodes. In the middle left column, the electrical settings used to produce the fields F displayed with an unfolded contact array where blue refers to contacts that are grounded, and red displays cathodes are shown. In the middle right column it is shown how grounding can be applied during steering stimulation. The focusing effect is gradually increased from top to bottom. In the right column it is shown that the electrical settings used to produce the fields displayed with an unfolded contact array.

## Claims

1. A system (10) for planning and/or providing a therapy for neural applications, especially for neurostimulation and/or neurorecording applications, comprising at least one lead (300), the lead (300) having a plurality of electrodes (132), at least one electrode (132) being capable to form an active contact (134) with a first potential, wherein the system (10) comprises at least one electrode potential adjusting means (400) which is configured such that at least one selected electrode (136) at (a) varying distance(s) from the active contact (134) may be provided with a second potential different from the first potential of the active contact (134) for the purpose of focusing the stimulation field (F) provided by the active contact (134).

2. The system (10) according to claim 1,
**characterized in that**
the plurality of electrodes (132) forms a complex geometrical array and/or that the plurality of electrodes (132) is arranged circumferentially around at least one section of the lead, especially around a section next to the distal tip end of the lead (300), and/or that the electrode potential adjusting means (400) is configured such that at least one selected electrode (136) at (a) varying distance(s) from the active contact (134) may be provided with a second potential different from the first potential of the active contact (134) for the purpose of focusing the stimulation field (F) provided by the active contact (134) automatically and/or semi-automatically.

3. The system (10) according to claim 1 or 2,
**characterized in that**
the system (10) is configured such that the second potential may be provided by providing and/or setting at least one electrode (136) which is not a part of the active contact (134) (to) a second polarity different from the first polarity of the active contact (134) and/or by grounding at least one electrode (136) which is not a part of the active contact (134), wherein especially the electrode potential adjusting means (400) is configured such that the second potential may be provided by providing and/or setting at least one electrode (136) which is not a part of the active contact (134) (to) a second polarity different from the first polarity of the active contact (134) and/or by grounding at least one electrode (136) which is not a part of the active contact (134).

4. The system (10) according to one of the preceding claims, **characterized in that**
the electrode potential adjusting means (400) is configured such that at least one electrode (136) at (a) varying distance(s) from the active contact (134) may be provided with at least one second potential different from the potential of the active contact (134) for the purpose of focusing the stimulation field provided by the active contact (134) in a first manner and that at least one electrode (136) at (a) varying distance(s) from the active contact (134) may be provided with at least one third potential different from the potential of the active contact (134) for the purpose of focusing the stimulation field provided by the active contact (134) in a second manner.

5. The system (10) according to claim 4,
**characterized in that** the second potential different from the potential of the active contact (134) leads higher amount of focussing by setting at least one electrode (136) next and/or adjacent from the active contact (134) and the electrode(s) (136) set for the second manner and that the third potential different from the potential of the active contact (134) leads lower amount of focussing by setting at least one electrode (136) further away from the active contact (134) and the electrode(s) (136) set for the first manner.

6. The system (10) according to one of the preceding claims, **characterized in that**
the electrode potential adjusting means (400) is configured such that the Euclidian distance between at least one of the electrodes not belonging to the active contact (134) is calculated, wherein especially the Euclidian distance between at least one of the electrodes not belonging to the active contact (134) is calculated by means of a distance calculation.

7. The system (10) according to one of the preceding claims, **characterized in that**
the electrode potential adjusting means (400) is configured such that the total surface area of all electrodes (132; 136) with a potential different from the potential of the active contact (134) is related to the amount of delivered current and the number of electrodes (132; 136) with a potential different from the potential of the active contact (134) is adjusted according to a predetermined safety threshold value such that the charge density safety limit is not reached when focused stimulation is turned on.

8. The system (10) according to one of the preceding claims, **characterized in that**
the electrode potential adjusting means (400) is configured such that the potential different from the potential of the active contact (134) may be gradually and/or stepwise decreased and/or increased.

9. A system for neural applications (100), especially a system for neurostimulation and/or neurorecording applications (100), for instance a deep brain stimulation system (100), the system for neural applications (100) comprising at least one system (10) for planning and/or providing a therapy for neural applications according to one of the preceding claims.

10. A method for focusing the stimulation field provided by an active contact (134) of a lead (300), wherein the lead (300) comprises a plurality of electrodes (132), at least one electrode (132) being capable to form an active contact (134), wherein the method comprises at least the step of providing electrodes (136) at varying distances from the active contact (134) with a second potential different from the first potential of the active contact (134) for the purpose of focusing the stimulation field (F) provided by the active contact (134).

11. The method of claim 10,
**characterized in that**
the different potential as the active contact (134) is provided by providing and/or setting at least one electrode (136) which is not a part of the active contact (134) (to) a second polarity different from the first polarity of the active contact (134) and/or by grounding at least one electrode (136) which is not a part of the active contact (134).

12. The method of claim 10 or 11,
**characterized in that**
at least one electrode (136) at (a) varying distance(s) from the active contact (134) may be provided with at least one second potential different from the potential of the active contact (134) for the purpose of focusing the stimulation field provided by the active contact (134) in a first manner and that at least one electrode (136) at (a) varying distance(s) from the active contact (134) may be provided with at least one third potential different from the potential of the active contact (134) for the purpose of focusing the stimulation field provided by the active contact (134) in a second manner, wherein in particular the second potential different from the potential of the active contact (134) leads to a higher amount of focussing by setting at least one electrode (136) next and/or adjacent from the active contact (134) and the electrode(s) (136) set for the second manner and that the third potential different from the potential of the active contact (134) leads to a lower amount of focussing by setting at least one electrode (136) further away from the active contact (134) and the electrode(s) (136) set for the first manner.

13. The method according to one of claims 10 to 12, **characterized in that**
the Euclidian distance between at least one of the electrodes not belonging to the active contact (134) is calculated, wherein especially the Euclidian distance between at least one of the electrodes not belonging to the active contact (134) is calculated by means of a distance calculation.

14. The method according to one of claims 10 to 13, **characterized in that**
the total surface area of all electrodes (132) with a potential different from the potential of the active contact (134) is related to the amount of delivered current and the number of electrodes (132) with a potential different from the potential of the active contact (134) is adjusted according to a predetermined safety threshold value such that the charge density safety limit is not reached when focused stimulation is turned on.

15. The method according to one of claims 11 to 14, **characterized in that**
the method is conducted with at least one system (10) for planning and/or providing a therapy for neural applications according to one of claims 1 to 9 and/or a system for neural applications (100) according to claim 10.
